## Europäisches Patentamt

## European Patent Office

## Office européen des brevets

(19)

(11) Veröffentlichungsnummer: **0 156 164**
**B1**

(12) **EUROPÄISCHE PATENTSCHRIFT**

(45) Veröffentlichungstag der Patentschrift:
**17.11.88**

(51) Int. Cl.⁴: **A 61 F 5/44**

(21) Anmeldenummer: **85101868.9**

(22) Anmeldetag: **21.02.85**

(54) Entlüftungsventil für einen Colostomiebeutel oder dgl.

(30) Priorität: **15.03.84 DE 3409527**

(43) Veröffentlichungstag der Anmeldung:
**02.10.85 Patentblatt 85/40**

(45) Bekanntmachung des Hinweises auf die Patenterteilung:
**17.11.88 Patentblatt 88/46**

(84) Benannte Vertragsstaaten:
**AT BE CH DE FR GB IT LI NL SE**

(56) Entgegenhaltungen:
**WO - A - 83/01377**
**DK - C - 102 058**
**GB - A - 2 036 564**
**GB - A - 2 083 760**
**US - A - 3 865 109**
**US - A - 3 952 727**

(73) Patentinhaber: **Beiersdorf Aktiengesellschaft,
Unnastrasse 48, D-2000 Hamburg 20 (DE)**

(72) Erfinder: **Sinnen, Marike, Fröbelstrasse 7,
D-2080 Pinneberg (DE)**
Erfinder: **Detering, Jürgen, Jenerseitedeich 126,
D-2102 Hamburg 93 (DE)**
Erfinder: **Meitzner, Uwe, Boberger Strasse 17,
D-2000 Hamburg 74 (DE)**

ACTORUM AG

**Beschreibung**

Die Erfindung betrifft ein Entlüftungsventil für medizinische Auffangbeutel, insbesondere Colostomiebeutel, mit einer Ventilöffnung und zwei Klappen, das an der Außenwandung des Beutels über einer Entlüftungsöffnung anzubringen ist.

Es ist inzwischen üblich geworden, Colostomiebeutel, die zur Versorgung eines künstlichen Darmausganges dienen und Darminhalt sowie -Gase aufnehmen sollen, mit einem integrierten oder mit einem von den Betroffenen selbst auf den Beutel aufzubringenden Entlüftungsfilter in den verschiedensten Ausführungsformen zu versehen. Durch derartige Filter, die in der Regel mit desodorierenden Substanzen wie Aktivkohle ausgerüstet sind, können die Darmgase entweichen, die anderenfalls den Beutel unnötig aufblähen.

Aus der GB-A-2036564 ist ein solcher integrierter Filter bekannt, der in einer Ausnehmung in der Beutelwand angeordnet ist, mit einer Gasauslaßöffnung in dem nach außen versetzten Wandflächenabschnitt, die so groß bemessen ist, daß sie den Strom des auszuleitenden Gases behinderungsfrei durchläßt. Vorzugsweise ist die Gasaustrittsöffnung als S-förmiger Schlitz ausgebildet, dessen Ränder sich bei geringfügigem Druckanstieg im Beutel voneinander lösen und das Gas entweichen läßt.

Aus der US-A-3865109 ist ein ähnliches Ventil zum Entlüften von Colostomiebeuteln bekannt, jedoch ohne Filtervorrichtung für die Gase. Es besteht aus einem laminierten Stückchen Flachmaterial, das auf dem Beutel aufgeklebt und mit einem Schlitz versehen ist, der auch durch die darunterliegende Beutelwand hindurchgeht. Durch diese Öffnung können die Darmgase entweichen. Anschließend sollen sich durch die Rückstellfähigkeit des Materials die beiden Hälften wieder aneinanderlegen und die Öffnung verschließen.

Die DK-C-102058 betrifft einen Ventilsack mit einer Einfüllöffnung und einem Verschluß für diese Öffnung, der aus einer Tasche auf der einen Seite und einer Lasche auf der anderen Seite der Öffnung besteht. Nach dem Füllen des Sackes wird die Lasche in die Tasche gesteckt und dadurch die Öffnung verschlossen.

Bei den bekannten Entlüftungsvorrichtungen hat sich als nachteilig herausgestellt, daß durch sie die gesamte Luft aus dem Beutel entweicht. Dies hat zur Folge, daß durch die Peristaltik des Darms ein gewisser Unterdruck im Beutel entsteht, was nicht nur den reibungslosen Transport der Ausscheidungen in den Beutel hinein behindert, sondern auch bewirken kann, daß die dann auf dem Stoma aufliegende, dem Körper abgewandte Beutelfolienseite in die empfindliche Stomaöffnung hineingezogen wird. Für den Betroffenen kann das unangenehm und zum Teil sehr schmerzhaft sein.

Aufgabe der Erfindung war es deshalb, eine Vorrichtung mit Ventilwirkung zu entwickeln, die zwar eine gute Entlüftung des Beutels ermöglicht, aber gleichzeitig dafür sorgt, daß im Beutel kein Unterdruck entsteht und damit diese Nachteile nicht auftreten.

Die erfindungsgemäße Lösung des Problems besteht darin, die Entlüftung durch ein regulierendes Ventil so abzubremsen, daß im Beutel ein leichter Überdruck von etwa 8–20 mm WS (Wassersäule), d.h. ein Restluftpolster, erhalten bleibt. Erreichen läßt sich dieser Effekt bei einem Entlüftungsventil für Beutel der anfangs genannten Art durch eine Basisplatte mit Ventilloch und einer an der Basisplatte befestigten klappenartig ausgebildeten Membran-Folie, die das Ventilloch überdeckt, sowie einer zweiten klappenartigen Folie, die gegenüber der ersten Folie an der Basisplatte befestigt ist und die Membran-Folie ganz oder teilweise überdeckt, wobei die Klappen das Ventilloch bis zu einem geringen Überdruck von etwa 8 bis 20 mm WS im Beutel verschlossen halten.

Das erfindungsgemässe Ventil funktioniert im Prinzip so, daß es sich bei Überdruck im Beutel öffnet und das Gas weitgehend entweichen läßt. Die beiden Klappen bleiben dabei in überdeckender Lage, sie werden bei Gasaustritt nur etwas angehoben, so daß das Gas – in erster Linie seitlich – entweichen kann. Ist der Überdruck im Beutel auf 8–20 mm WS abgesunken, vermag er durch den Gegendruck der Deckfolie auf die Membranfolie die Klappen nicht mehr anzuheben und diese halten die Öffnung geschlossen. Ein derartiger Überdruck ist gerade erwünscht, da er den Beutel noch nicht störend aufbläht, aber wie ein Polster wirkt, und ein Aufliegen der Beutelfolie auf das Stoma oder gar ein Einziehen in dieses verhindert.

Als Material für die Ventilklappen können eine ganze Reihe von folienartigen Materialien, die für beide Klappen gleich oder verschieden sein können, eingesetzt werden. Insbesondere eignen sich Folien auf Kunststoff-Basis wie Weich- oder Hart-PVC, Polyester, Polyethylen oder Polypropylen. Aber auch dünne Metallfolien oder Papiere lassen sich verwenden, wenn sie nur die passende Geschmeidigkeit und gleichzeitig Festigkeit aufweisen, um durch ihr Zusammenwirken den oben beschriebenen Effekt des Schliessens bei geringem Überdruck zu ergeben. Auch laminierte Produkte aus mehreren gleichen oder verschiedenen Folien sind möglich.

Die Dicke der Membranfolie kann, jeweils abgestimmt auf die sonstigen Materialeigenschaften der Folie, d.h. insbesondere ihre Steifheit, sich in dem Bereich von etwa 15–200 μm, vorzugsweise etwa 40–150 μm, bewegen. Die Deckfolie, welche die Membranfolie bei noch geringem Überdruck vom Inneren des Beutels her bereits runterdrücken, d.h. schließen, muß, kann etwas dicker bemessen sein, etwa 20–250 μm.

Die beiden Ventilklappen sind jeweils in einem schmalen Bereich am linken bzw. rechten äußeren Rand der Basisplatte befestigt, vorzugsweise durch eine Klebemassenschicht oder auch eine Verschweißungszone. Mit ihrem in der Regel größeren Teil sind sie dann klappenartig beweglich. Die untere Membranfolie muß zumindest das Ventilloch bedecken, die darüberliegende Deck- oder Zuhaltefolie überlappt diese ganz oder teilweise. Als notwendig hat es sich erwiesen, daß der überlappende Teil nicht zu klein gewählt wird, da sonst die Gefahr besteht, daß bei starkem Gasaustritt oder durch Bewegung der darüber befindlichen Kleidung die Deckfolie unter die Membranfolie rutscht und damit das Ventil seine Wirksamkeit verliert. Besonders vorteilhaft ist es deshalb, das Ventil symmetrisch zu gestalten, so daß Membran- und Deckklappe sich gegenseitig in ihrer Funktion austauschen können und dieser negative Effekt nicht auftritt.

Die Basisplatte mit dem Ventilloch kann aus den verschiedensten Materialien bestehen, vorzugsweise einem Flachmaterial wie einer Kunststoffoder Metallfolie, Papier, Vlies oder Schaumstoff, wobei auch Laminate aus einem oder mehreren dieser Produkte möglich sind. Zur einfachen Befestigung des Ventils am Beutel ist die Basisplatte an ihrer Unterseite mit einer Selbstklebemassenschicht ausgerüstet, die bis zur Verwendung der Vorrichtung mit einem Schutzblatt abgedeckt ist.

Das erfindungsgemäße Entlüftungsventil besteht in seiner einfachsten Ausführungsform aus einer folienartigen Basisplatte und den beiden auf dieser befestigten Ventilklappen. In dieser Form wird es bei außen auf dem Beutel angebrachten Entlüftungsfiltern direkt auf deren Entlüftungsloch geklebt, bei im Beutel, d.h. an der Beutelinnenwand, angebrachten Filtern wird es deckungsgleich auf das in der dem Körper abgewandten Beutelaußenwand angebrachten Entlüftungsloch geklebt. Jedoch sind auch integrierte Systeme möglich, wo die Basisplatte gleich als desodorierende Filterplatte, beispielsweise aus mit Aktivkohle getränktem Schaumstoff oder Vlies, ausgebildet ist und vom Gas vor seinem Austritt zu durchströmen ist. Auch andere Filterformen als plattenförmige lassen sich selbstverständlich mit dem Regulierventil ausrüsten.

Die Entlüftungsöffnung in der Basisplatte ist üblicherweise ein kleines Loch, jedoch besteht auch die Möglichkeit, die Basisplatte ganz oder teilweise porös und gasdurchlässig, beispielsweise durch Perforation, auszugestalten.

Eine beispielsweise Ausführungsform des erfindungsgemäßen Ventils läßt sich wie folgt herstellen:

Beispiel 1

Als Basisplatte wird eine kleine runde Scheibe einer 190 μm starken Polyesterfolie eingesetzt. Der Durchmesser der runden Platte beträgt 20 mm, mittig befindet sich ein Öffnungsloch mit einem Durchmesser von 7 mm. Auf einer Seite wird die Platte mit einer üblichen Acrylatselbstklebemasse in einer Stärke von etwa 30 g/m² beschichtet und mit einem silikonisierten Papier abgedeckt.

Auf die nichtklebende Seite dieser Basisplatte wird die das Loch verschließende klappenartige Membranfolie wie folgt angebracht: Auf eine 25 μm starke 19 mm breite und einseitig mit einer Acrylatklebemasse (30 g/m²) beschichtete Polyesterfolie wird eine 10 mm breite 25 μm starke nichtklebende Polyesterfolie an einem Rand kantenbündig aufkaschiert. Der verbleibende freiliegende, einseitig klebende Teil dieses Verbundproduktes wird so auf die Basisplatte aufgebracht, daß der nichtklebende Teil die Öffnung in der Basisplatte bedeckt (verschliesst). Überstehende Ränder werden weggestanzt.

Die auf diese Weise aufgebrachte Klappe wird durch eine in Gegenüberstellung auf der Basisplatte anzubringende Klappe, die sowohl kleiner, als auch größer oder gleichgroß sein kann, verschlossen. Die Einsatzmaterialien und die Herstellung des Verbundproduktes für diese Deckklappe sind identisch mit der Membranklappe, lediglich der nichtklebende Teil ist jetzt nur 5 mm breit. d.h. in diesem Fall, daß die Deckklappe schmaler ist als die Membranklappe.

Wird ein derartiges Ventil auf die Entlüftungsöffnung eines gebräuchlichen Colostomiebeutels aufgebracht und dieser mit Gas gefüllt, heben sich die Klappen und die Gase treten ins Freie. Nach Absinken des Druckes im Beutelinnern auf etwa 7–8 mm WS (Überdr.)(etwa 70–80 Pa) drückt die obere Klappe die darunterliegende wieder zu, und es verbleibt eine geringe Menge Luft als Polster im Beutel.

In gleicher Weise wurden eine Reihe weiterer Entlüftungsventile hergestellt aus unterschiedlichen Materialien und deren Verhalten im Test geprüft.

Beispiele 2–10

Als Deckfolie wurde eine Polyesterfolie wie in Beispiel 1 beschrieben eingesetzt, die Membranfolie wurde nach Material und Dicke variiert und der Restüberdruck im Beutel nach Gasentleerung mit jeweils 3 Messungen bestimmt.

| Beispiel Nr. | Membranfolie | Restüberdruck im Beutel |
|---|---|---|
| 2 | PVC (hart), 40 μm | 13 mm WS |
| | | 11 mm WS |
| | | 11 mm WS |
| 3 | PVC (hart), 66 μm | 10 mm WS |
| | | 9 mm WS |
| | | 10 mm WS |
| 4 | PVC (hart), 125 μm | 12 mm WS |
| | | 11 mm WS |
| | | 11 mm WS |
| 5 | PVC (weich), 122 μm | 9 mm WS |
| | | 10 mm WS |
| | | 10 mm WS |
| 6 | PVC (weich), 85 μm | 13 mm WS |
| | | 12 mm WS |
| | | 14 mm WS |
| 7 | Polyäthylen, 105 μm | 12 mm WS |
| | | 12 mm WS |
| | | 9 mm WS |
| 8 | Polyäthylen, 160 μm | 10 mm WS |
| | | 10 mm WS |
| | | 11 mm WS |

| Beispiel Nr. | Membranfolie | Restüberdruck im Beutel |
|---|---|---|
| 9 | Polypropylen, 60 μm | 11 mm WS |
| | | 11 mm WS |
| | | 10 mm WS |
| 10 | Polypropylen, 70 μm | 10 mm WS |
| | | 11 mm WS |
| | | 12 mm WS |

Beispiele 11–13

Als Membranfolie wurde eine Polyesterfolie wie in Beispiel 1 beschrieben eingesetzt, die Deckfolie wurde variiert, indem auf eine 25 μm dicke Polyesterfolie eine zweite Polyesterfolie verschiedener Dicke geklebt wurde. Dei Acrylat-Klebemassenschicht dazwischen war wieder 30 g/m² stark. Der Restüberdruck im Beutel nach Gasentleerung wurde mit jeweils 4 Messungen bestimmt.

| Beispiel Nr. | Deckfolie | Restüberdruck im Beutel |
|---|---|---|
| 11 | Verbundfolie aus einer 25 μm und einer 36 μm dicken Polyesterfolie | 15 mm WS<br>13 mm WS<br>11 mm WS<br>11 mm WS |
| 12 | Verbundfolie aus einer 25 μm und einer 10 μm dicken Polyesterfolie | 9 mm WS<br>9 mm WS<br>9 mm WS<br>9 mm WS |

| Beispiel Nr. | Deckfolie | Restüberdruck im Beutel |
|---|---|---|
| 13 | Verbundfolie aus einer 25 μm und einer 75 μm dicken Polyesterfolie | 12 mm WS<br>11 mm WS<br>17 mm WS<br>16 mm WS |

Wie die Beispiele zeigen, läßt sich durch Dicke und Art der Membran- bzw. Deckfolie der verbleibende Restüberdruck im Beutel in gewissen Grenzen variieren und damit auch individuell an die Erfordernisse des Benutzers anpassen, denn je nach Ausbildung des Stomas kann es wünschenswert sein, ein etwas stärkeres Aufblähen des Beutels einzustellen, damit die Beutelwand nicht am empfindlichen Stoma anliegt.

Besonders vorteilhaft hat sich ein Entlüftungsventil gemäß Beispiel 6 erwiesen aus einer 85 μm dicken Weich-PVC-Folie als Membranfolie und einer Verbund-Polyester-Deckfolie gemäß Beispiel 1. Die Ventilklappenfolien haften – wohl aufgrund einer gewissen elektrostatischen Aufladung – schmiegsam aufeinander und rutschen deshalb auch bei stärkerer Bewegung des Benutzers und dem dadurch erzeugten Scheuern der darüber befindlichen Kleidung nicht auseinander.

Die Erfindung wird nachstehend anhand des in den Zeichnungen dargestellten Ausführungsbeispiels näher erläutert.

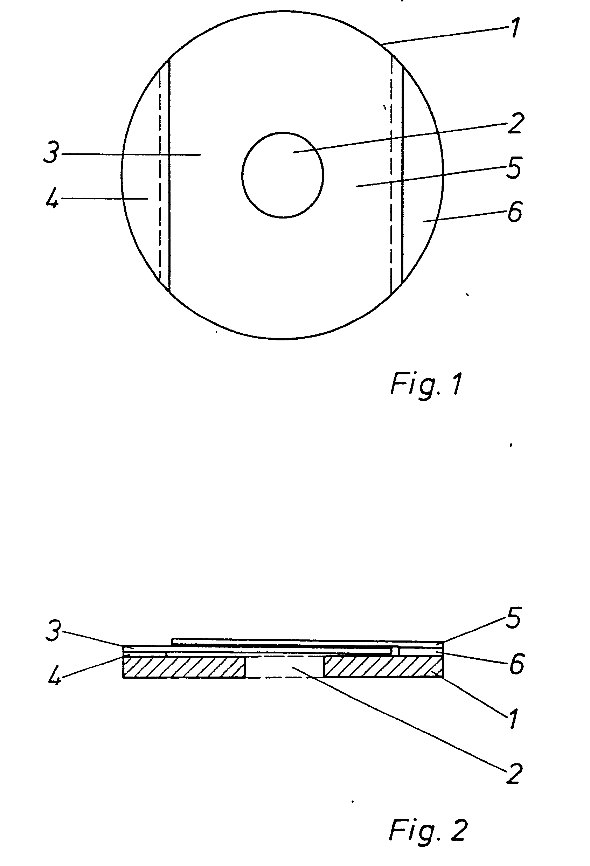

Die Abbildungen 1 und 2 zeigen im vergrößerten Maßstab das erfindungsgemäße Ventil von der Oberseite und im Querschnitt. Dabei bedeuten 1 die Basisplatte, 2 das Ventilloch, 3 die Membranfolie mit der Klebeschicht 4 und 5 die darüberliegende Deckfolie mit der Klebeschicht 6.

## Patentansprüche

1. Entlüftungsventil für medizinische Auffangbeutel, insbesondere Colostomiebeutel, mit einer Ventilöffnung und zwei Verschlußteilen, gekennzeichnet durch eine Basisplatte (1) mit Ventilloch (2) und einer an der Basisplatte (1) befestigten klappenartig ausgebildeten Membran-Folie (3), die das Ventilloch (2) überdeckt, sowie einer zweiten klappenartigen Folie (5), die gegenüber der ersten Folie (3) an der Basisplatte (1) befestigt ist und die Membran-Folie (3) ganz oder teilweise überdeckt, wobei das Ventil den Druck im Beutel in der Weise reguliert, daß die Klappen (3, 5) das Ventilloch (2) bis zu einem Überdruck von etwa 8 bis 20 mm WS (Wassersäule) im Beutel verschlossen halten:

2. Entlüftungsventil nach Anspruch 1, dadurch gekennzeichnet, daß die Membranfolie (3) und die Deckfolie (5) aus einer Kunststoff-Folie, insbesondere aus Weich- oder Hart-PVC, Polyester, Polyethylen oder Polypropylen bestehen.

3. Entlüftungsventil nach Anspruch 1 oder 2, dadurch gekennzeichnet, daß die Membranfolie (3) 15–200 μm, vorzugsweise 40–150 μm, dick ist.

4. Entlüftungsventil nach Anspruch 1 oder 2, dadurch gekennzeichnet, daß die Deckfolie (5) 20–250 μm dick ist.

5. Entlüftungsventil nach Anspruch 1, dadurch gekennzeichnet, daß die Membranfolie (3) aus einer Weich-PVC-Folie und die Deckfolie (5) aus einer Polyesterfolie besteht.

6. Entlüftungsventil nach einem oder mehreren der Ansprüche 1–5, dadurch gekennzeichnet, daß die Basisplatte (1) aus einer Kunststoff- oder Metallfolie, Papier, Vlies oder Schaumstoff besteht und gegebenenfalls an ihrer Unterseite mit einer Selbstklebemasse beschichtet ist.

7. Entlüftungsventil nach einem oder mehreren der Ansprüche 1–6, dadurch gekennzeichnet, daß die Basisplatte (1) als Filterplatte ausgebildet ist und desodorierende Substanzen, vorzugsweise Aktivkohle, enthält.

8. Entlüftungsventil nach einem oder mehreren der Ansprüche 1–7, dadurch gekennzeichnet, daß anstelle eines einzigen Ventilloches (2) die Basisplatte (1) als Ganzes oder teilweise gasdurchlässig bzw. perforiert ausgebildet ist.

## Claims

1. Air-release valve for medical collecting bags, in particular colostomy bags, with a valve opening and two sealing parts, characterized by a base plate (1) with a valve hole (2) and a membrane film (3) which is made in the form of a flap and is fixed to the base plate (1) and which covers the valve hole (2), and with a second flaplike film (5) which is fixed to the base plate (1) opposite the first film (3) and wholly or partially covers the membrane film (3), the valve regulating the pressure in the bag in such a way thet the flaps (3, 5) hold the valve hole (2) sealed up to a positive pressure of about 8 to 20 mm water gauge in the bag.

2. Air-release valve according to Claim 1, characterized in that the membrane film (3) and the cover film (5) consist of a plastic film, in particular of soft or rigid PVC, polyester, polyethylene or polypropylene.

3. Air-release valve according to Claim 1 or 2, characterized in that the membrane film (3) is 15–200 μm and preferably 40–150 μm thick.

4. Air-release valve according to Claim 1 or 2, characterized in that the cover film (5) is 20–250 µm thick.

5. Air-release valve according to Claim 1, characterized in that the membrane film (3) consists of a soft PVC film and the cover film (5) consists of a polyester film.

6. Air-release valve according to one or more of Claims 1–5, characterized in that the base plate (1) consists of a plastic film or metal foil, paper, nonwoven or foam and, if appropriate, is coated on its underside with a self-adhesive composition.

7. Air-release valve according to one or more of Claims 1–6, characterized in that the base plate (1) is made in the form of a filter plate and contains deodorizing substances, preferably active charcoal.

8. Air-release valve according to one or more of Claims 1–7, characterized in that, instead of the single valve hole (2), the base plate (1), as a whole or partially, is made gas-permeable or perforated.

**Revendications**

1. Valve de désaérage pour une poche de collecte médicale, en particulier pour une poche de colostomie, avec une ouverture de valve et deux pièces de fermeture, caractérisée par une plaque de base (1) avec un trou de valve (2) et une feuille-membrane (3) formant clapet fixée à la plaque de base (1), qui recouvre le trou de valve (2), ainsi qu'avec, une seconde feuille (5) formant clapet, qui est fixée à la plaque de base (1) en face de la première feuille (3) et qui recouvre entièrement ou partiellement la feuille-membrane (3), la valve réglant la pression dans la poche de telle sorte que les clapets (3, 5) maintiennent le trou de valve (2) fermé jusqu'à une surpression d'environ 8 à 20 mm CE (collonne d'eau) dans la poche.

2. Valve de désaérage suivant la revendication 1, caractérisée en ce que la feuille-membrane (3) et la feuille de couverture (5) sont constituées par une feuille en matière plastique, en particulier en PVC plastifié ou rigide, en polyester, en polyéthylène ou en polypropylène.

3. Valve de désaérage suivant la revendication 1 ou 2, caractérisée en ce que la feuille-membrane (3) a une épaisseur de 15–200 µm, de préférence de 40–150 µm.

4. Valve de désaérage suivant la revendication 1 ou 2, caractérisée en ce que la feuille de couverture (5) a une épaisseur de 20–250 µm.

5. Valve de désaérage suivant la revendication 1, caractérisée en ce que la feuille-membrane (3) se compose d'une feuille de PVC plastifié et la feuille de couverture (5) d'une feuille de polyester.

6. Valve de désaérage suivant une ou plusieurs des revendications 1–5, caractérisée en ce que la plaque de base (1) se compose d'une feuille de matière plastique ou de métal, de papier, de feutre ou de mousse et en ce qu'elle est éventuellement garnie d'une couche autocollante sur sa face inférieure.

7. Valve de désaérage suivant une ou plusieurs des revendications 1 à 6, caractérisée en ce que la plaque de base (1) est une plaque filtrante et contient des substances désodorisantes, de préférance du charbon actif.

8. Valve de désaérage suivant une ou plusieurs des revendications 1–7, caractérisée en ce qu'au lieu d'un seul trou de valve (2), la plaque de base (1) est entièrement ou partiellement perméable aux gaz, respectivement perforée.

Fig. 1

Fig. 2